# FASCICULE DE BREVET EUROPEEN

(11) **EP 1 786 282 B1**
(45) Date de publication et mention de la délivrance du brevet: **23.01.2008**
(21) Numéro de dépôt: 05776468.0
(22) Date de dépôt: 09.06.2005
(51) Int. Cl.: A23L 3/04

(54) **PROCEDE DE PASTEURISATION OU DE STERILISATION ET SON DISPOSITIF DE MISE EN OEUVRE**
PASTEURISATIONS- BZW. STERILISATIONSVERFAHREN UND VORRICHTUNG ZUR AUSFÜHRUNG DIESES VERFAHRENS
PASTEURISATION OR STERILISATION METHOD AND DEVICE FOR CARRYING OUT SAME

(30) Priorité: 14.06.2004 FR 0451173
(43) Date de publication de la demande: 23.05.2007
(73) Titulaire: Camu, Patrice, 67700 Saverne (FR)
(72) Inventeur: Camu, Patrice, 67700 Saverne (FR)
(74) Mandataire: Rhein, Alain
(86) Numéro de dépôt international: PCT/FR2005/050433
(87) Numéro de publication internationale: WO 2006/000728

(56) Documents cités:
- DE-C- 745 426
- US-A- 3 340 791
- US-A- 3 407 721

## Description

La présente invention concerne un procédé et son dispositif de mise en oeuvre pour la pasteurisation ou la stérilisation de produits, en particulier sous emballage clos souple ou rigide.

La stérilisation et la pasteurisation ont pour but d'augmenter la durée de conservation des aliments. Ces opérations nécessitent une élévation de température qui est généralement suivie d'une phase de refroidissement du produit.

Au cours de ces opérations, l'élévation de température crée une augmentation de la pression à l'intérieur de l'emballage. Il est alors nécessaire de compenser cette pression interne par une pression appliquée sur l'extérieur de l'emballage.

Les procédés et dispositifs existants requièrent donc une enceinte maintenue sous pression lors des opérations de stérilisation, pasteurisation et refroidissement sont effectuées. Un dispositif existant est une enceinte dans laquelle les produits sont placés au travers de moyens d'entrée et/ou de sortie. Ladite enceinte est ensuite mise sous pression, l'augmentation de pression s'effectuant au fur et à mesure de l'élévation de température à l'intérieur de l'enceinte close. La température et la pression sont ensuite diminuées et les produits sortis de l'enceinte par les moyens d'entrée et/ou sortie. De tels dispositifs sont connus de l'état de la technique, comme les autoclaves.

L'inconvénient de ces dispositifs est l'aspect discontinu du procédé. Pour pallier cette discontinuité, une installation hydrostatique a été conçue pour offrir la possibilité de stériliser ou pasteuriser les produits en continu.

Le principe hydrostatique consiste à faire traverser les produits à stériliser ou à pasteuriser depuis le haut vers le bas d'une colonne de fluide hydrostatique de chauffage, la surface de la colonne de fluide étant ouverte à l'air libre, et par conséquent soumise à la pression atmosphérique. Au fur et à mesure de la descente du produit dans la colonne, ce dernier subit une pression croissante appliquée par le fluide, compensant l'augmentation interne de pression, due à l'élévation de température. Une pression maximale peut être appliquée en partie basse de la colonne et peut varier proportionnellement à la hauteur de liquide dans la colonne. Le produit doit ensuite être remonté sous pression décroissante tout en étant refroidit.

Une solution pour la mise en oeuvre de ce principe, consiste à placer une paroi médiane pour séparer en deux la colonne de fluide de chauffage, à la manière d'un siphon, formant ainsi un circuit au sein de la colonne. Les produits sont alors acheminés depuis une entrée située en haut de la colonne d'un côté de la paroi vers le bas, subissant une pression croissante, puis du bas vers une sortie située en haut de la colonne de l'autre côté de la paroi, subissant une pression décroissante.

Pour obtenir une pression suffisante par rapport à la température de stérilisation ou de pasteurisation, la hauteur des installations serait de l'ordre de la dizaine de mètres et donc difficile à mettre en place. De plus, la température du liquide d'une colonne de chauffage peut difficilement varier depuis le haut vers le bas de la colonne et les produits nécessitent une phase de refroidissement sous pression.

Pour pallier ces inconvénients, il a été imaginé une installation sous la forme d'une enceinte comprenant un compartiment de chauffage hydrostatique séparé d'un compartiment de refroidissement hydrostatique par un compartiment intermédiaire de mise sous pression, cette séparation étant nécessaire pour éviter les échanges et les dissipations thermiques entre les compartiments de chauffage et de refroidissement.

Les compartiments de chauffage et de refroidissement sont chacun constitués d'au moins une colonne de fluide hydrostatique comme précédemment décrite. Dans le cas d'un compartiment comportant plusieurs colonnes consécutives, la pression, correspondant à la hauteur de liquide d'une première colonne, se répercute sur la colonne suivante et ainsi de suite depuis l'extérieur de l'enceinte, à la pression atmosphérique, vers le compartiment intermédiaire.

Le compartiment intermédiaire de mise sous pression sert d'isolant thermique entre les deux autres compartiments hydrostatiques et comporte des moyens de mise sous pression du compartiment intermédiaire.

Sans application de pression, les niveaux sont équilibrés de part et d'autre de la paroi médiane de chaque colonne. L'augmentation de pression à l'intérieur du compartiment intermédiaire impose une pression sur les surfaces de liquide des colonnes adjacentes audit compartiment. Lorsque la pression appliquée est suffisante, le niveau de la colonne baisse du côté de la cloison où la pression est appliquée et monte de l'autre côté.

La montée du niveau de liquide dans les colonnes crée une pression sur une colonne d'air séparant deux colonnes d'eau consécutives. Cette pression, une fois suffisamment élevée, fait baisser le niveau de liquide de la colonne suivante et monter le niveau situé de l'autre côté de la paroi médiane de cette colonne. Et ainsi de suite pour toute colonne adjacente.

La pression appliquée dans le compartiment intermédiaire se répercute donc sur chaque colonne suivante. La pression dans chaque colonne est fonction de la hauteur de liquide à déplacer dans ladite colonne. La pression appliquée à chaque colonne de liquide diminue d'une colonne à une autre dans le sens du centre vers les extrémités d'entrée et de sortie de l'enceinte, cette diminution provenant du volume total de liquide à déplacer toujours moindre au fur et à mesure que le nombre de colonnes diminue depuis le compartiment intermédiaire vers l'entrée ou la sortie de l'enceinte, à la pression atmosphérique.

La pression fournie par le compartiment intermédiaire peut être augmentée ou diminuée suivant les besoins, en fonction du type des produits et de leur température de stérilisation ou de pasteurisation. Toutefois, si cette pression dépasse une valeur maximale, le niveau de liquide des colonnes vient à déborder. La pression maximale applicable au sein du dispositif est donc majorée par la hauteur et le nombre de colonnes lors de la fabrication.

Les dispositifs connus de stérilisation ou pasteurisation en continu présentent donc l'inconvénient d'avoir une pression maximale fixée lors de la fabrication et par conséquent une température maximale de pasteurisation ou de stérilisation. Dans ces conditions, il se peut que pour la stérilisation ou la pasteurisation d'une gamme de produits, une grande majorité d'ente eux nécessite une installation dont le compartiment de chauffage comporterait un nombre de colonnes hydrostatiques limité, tandis que pour couvrir une minorité de produits, la contre-pression à appliquer serait telle qu'il faudrait augmenter sensiblement le nombre de ces colonnes, ayant pour conséquence une augmentation substantielle du coût de l'installation.

Dans le document US 3 340 791 est décrit un dispositif de stérilisation de produits, en particulier de conteneur en verre, qui s'affranchit néanmoins d'une partie de ces inconvénients. Pour ce faire, il comprend une enceinte sous pression dans laquelle circule un convoyeur sans fin acheminant des produits à stériliser depuis une chambre sous pression d'air élevée, vers un logement hydrostatique de chauffage, puis au travers d'un logement de stérilisation sous pression lui aussi, et vers un logement hydrostatique de refroidissement pour enfin retourner dans ladite chambre sous pression. A ce propos, ladite chambre comprend des moyens de chargement et de déchargement dudit convoyeur qui se présentent sous la forme d'une soupape rotative sous pression en entrée et en sortie de l'enceinte. Les logements hydrostatiques se présentent sous la forme d'un compartiment, rempli de liquide hydrostatique, disposé verticalement et dont une partie est allongée horizontalement pour chauffer ou refroidir à pression constante les produits. A ce propos, il convient de noter que ces logements hydrostatiques sont chauffés de manière à ce qu'à leurs extrémités hautes, débouchant dans le logement de stérilisation, le liquide soit chaud tandis qu'à leur extrémité basse, débouchant dans la chambre à pression d'air élevée, le liquide soit plus froid. De cette manière, les produits sont chauffés graduellement lors de leur passage dans le logement hydrostatique de chauffage et refroidis petit à petit lors de leur cheminement au travers du logement de refroidissement.

Même s'il permet de faire varier la pression au sein de l'enceinte en augmentant ou diminuant la pression de la chambre à pression élevée, accessible par des soupapes, en même temps que la pression du logement de stérilisation, ce dispositif est prévu pour la stérilisation d'emballage solide, comme une bouteille en verre et n'est pas adapté à des emballages souples ou partiellement souples. Ainsi il présente l'inconvénient d'imposer aux produits une pression trop élevée, la plus élevée dans toute l'enceinte, dès leur entrée dans la chambre de pression de sorte que des emballages souples peuvent être endommagés.

De plus, les dispositifs de l'état de la technique présente généralement l'inconvénient qu'une fois le procédé lancé, peu de possibilités de modification des paramètres sont possibles, comme la pression et les températures subies par les produits, mais plus particulièrement le temps de stérilisation, de chauffage et/ou de refroidissement.

L'invention a pour but de pallier les inconvénients de l'état de la technique, notamment, en offrant un dispositif capable de faire varier la pression, et donc la température, à l'intérieur de l'enceinte sans interrompre le cours du procédé de stérilisation ou de pasteurisation. De plus, l'invention permet de faire varier le temps de passage des produits à l'intérieur de l'enceinte, et donc la durée pendant laquelle ils subissent la pression et la température.

L'invention permet ainsi la stérilisation et la pasteurisation de produits dans une large gamme et comprenant des emballages rigides, souples ou partiellement souples.

Pour ce faire l'invention concerne un procédé de pasteurisation ou de stérilisation de produit, consistant à :
- transférer ledit produit depuis l'extérieur, à la pression atmosphérique, dans un compartiment d'entrée sous pression d'une enceinte au travers d'au moins un sas de mise sous pression ;
- charger ledit produit sur des moyens d'acheminement assurant leur transfert au travers de ladite enceinte depuis le compartiment d'entrée vers un compartiment de sortie ;
- chauffer le produit en le plongeant dans des colonnes hydrostatiques pour soumettre les produits à une pression proportionnelle à leur température ;
- faire traverser audit produit un compartiment intermédiaire de mise sous pression ;
- refroidir le produit en le plongeant dans des colonnes hydrostatiques pour soumettre les produits à une pression proportionnelle à leur température ;
- décharger ledit produit des moyens d'acheminement au niveau d'un compartiment de sortie sous pression ;
- transférer vers l'extérieur ledit produit depuis le compartiment de sortie au travers d'au moins un sas de mise à la pression atmosphérique.

En outre, le procédé consiste à faire varier le temps de passage du produit à l'intérieur de ladite enceinte en faisant varier la longueur de la trajectoire suivie de ces produits dans cette enceinte.

L'invention concerne aussi le dispositif de mise en oeuvre du procédé précédent, constitué d'une enceinte pourvue, d'une part, sous forme de colonnes hydrostatiques pour soumettre les produits à une pression proportionnelle à leur température, d'un compartiment de chauffage et d'un compartiment de refroidissement reliés par un compartiment intermédiaire de mise sous pression, ledit dispositif comportant encore des moyens d'acheminement des produits depuis une extrémité d'entrée vers une extrémité de sortie de l'enceinte, caractérisé par le fait que ladite enceinte comporte un compartiment d'entrée et un compartiment de sortie munis de moyens de mise sous pression et équipés respectivement, d'au moins un sas de mise sous pression des produits et d'au moins un sas de mise à la pression atmosphérique des produits.

Selon d'autres caractéristiques de l'invention, plusieurs sas sont raccordés en parallèle et/ou en série aux compartiments d'entrée et/ou de sortie.

En particulier, le ou les sas constituent, substantiellement, des moyens de chargement et de déchargement en produit des moyens d'acheminement à hauteur des compartiments d'entrée et de sortie.

En outre, le dispositif selon l'invention comporte des moyens de variation du temps de circulation d'un produit au travers de l'un quelconque des compartiments de l'enceinte.

Ces moyens de variation du temps de circulation consistent en des moyens de variation de la trajectoire parcourue par lesdits moyens d'acheminement entre les sas de chargement et de déchargement.

D'autres caractéristiques et avantages de l'invention ressortiront de la description détaillée qui va suivre des modes de réalisation non limitatifs de l'invention, en référence aux figures annexées dans lesquelles :
- la figure 1 représente un mode de réalisation du dispositif selon l'invention ;
- la figure 2 représente une enceinte comportant des sas de mise sous pression raccordés en parallèle au compartiment d'entrée de l'enceinte ;
- la figure 3 représente une enceinte comportant des sas de mise sous pression raccordés en série au compartiment d'entrée de l'enceinte ;
- la figure 4 représente une configuration en étoile d'un sas de mise sous pression ; et
- la figure 5 représente un schéma d'un mode particulier de réalisation de l'invention.

La présente invention concerne donc un procédé de stérilisation et de pasteurisation de produits et son dispositif de mise en oeuvre. Elle trouvera son application dans la stérilisation et la pasteurisation de produits contenus dans des emballages rigides, souples ou partiellement souples.

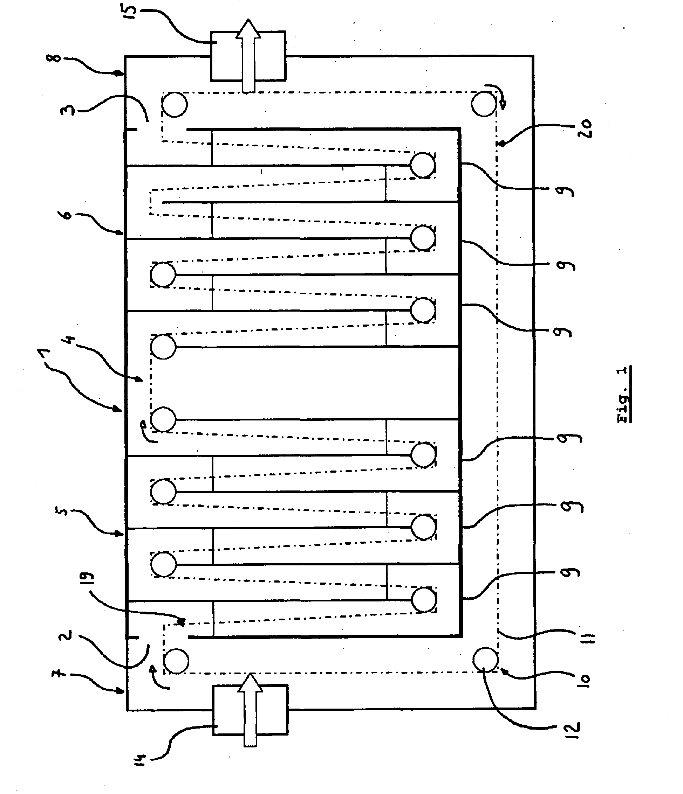

Un mode de réalisation du dispositif selon l'invention, visible sur la figure 1, comprend une enceinte 1 pourvue, sous forme d'au moins une colonne hydrostatique 9, d'un compartiment de chauffage 5 et d'un compartiment de refroidissement 6. Ceux-ci sont reliés par un compartiment intermédiaire 4, raccordé à des moyens de mise sous pression, non représentés. Les colonnes hydrostatiques 9, constituant lesdits compartiments de chauffage 5 et de refroidissement 6, renferment respectivement un liquide de chauffage et un liquide de refroidissement.

Le dispositif comporte encore un moyen d'acheminement 10 des produits au travers des différents compartiments 4,5,6, depuis une extrémité d'entrée 2 vers une extrémité de sortie 3 de l'enceinte 1. De tels moyens 10 peuvent se présenter sous la forme d'une chaîne de transport 11 sans fin, comportant des moyens de transports des produits, non représentés, sous la forme de nacelles, plateaux ou tout autre moyen approprié pour transporter les produits. Cette chaîne 11 est entraînée continuellement par des moyens d'entraînement, non représentés.

Ainsi, cette chaîne 11 traverse chacune des colonnes hydrostatiques 9 du compartiment de chauffage 5 et du compartiment de refroidissement 6, mais aussi le compartiment intermédiaire 4 en passant au-dessus de rouleaux de renvoi 12 adaptés. Par ailleurs, elle décrit un circuit de retour depuis l'extrémité de sortie 3 vers l'extrémité d'entrée 2.

De manière particulière à l'invention, l'enceinte 1 comporte, à son extrémité d'entrée 2 et à son extrémité de sortie 3, respectivement, un compartiment d'entrée 7 et un compartiment de sortie 8 raccordés chacun à des moyens de mise sous pression 13.

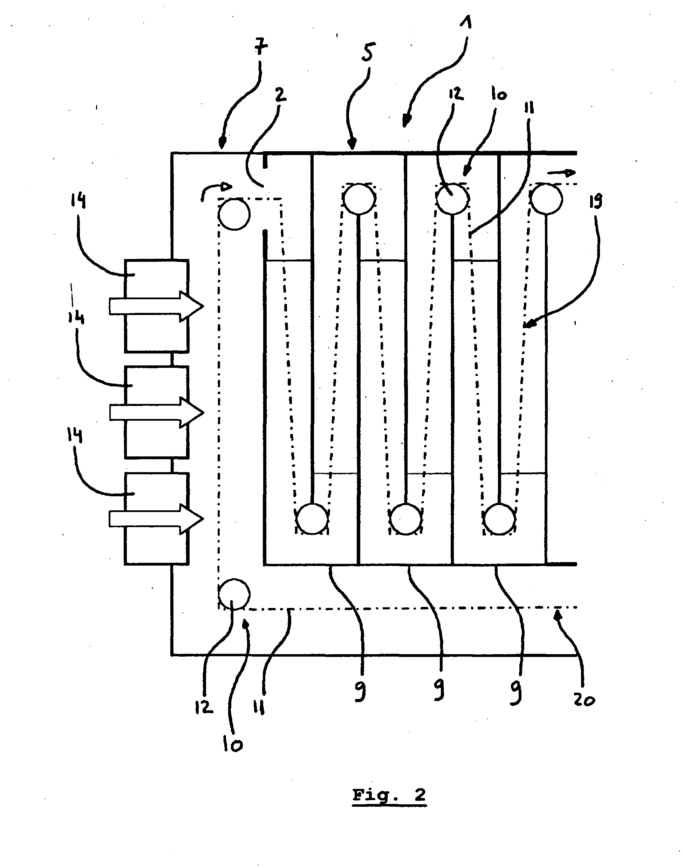

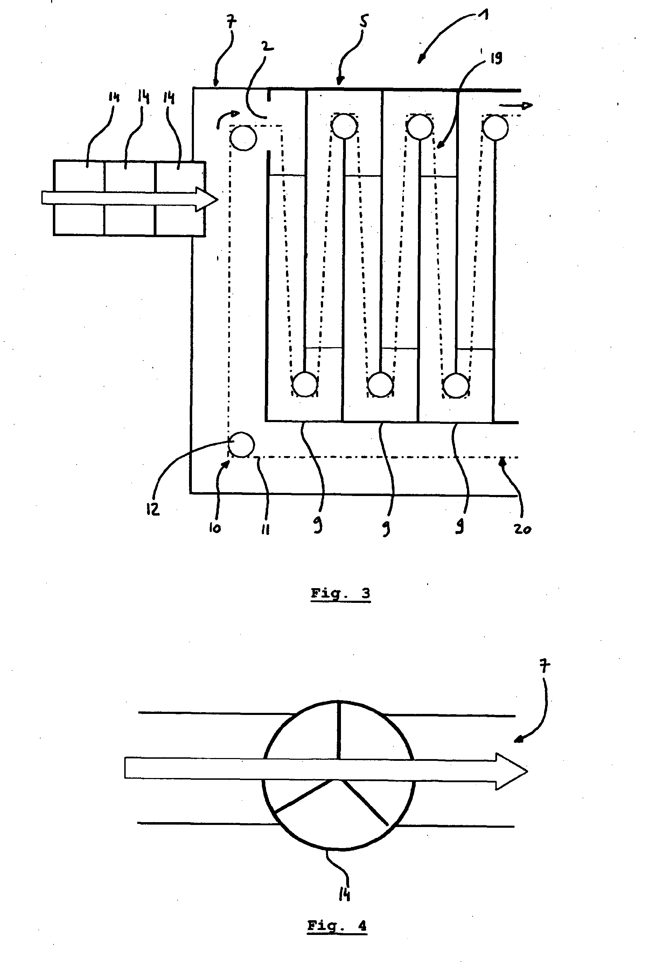

Les produits pénètrent dans le compartiment d'entrée 7 et sont extraits du compartiment de sortie 8 au travers, respectivement, d'au moins un sas de mise sous pression 14 et d'un sas de mise à la pression atmosphérique 15. Comme il est visible sur les figures 2 et 3, ces sas 14,15 peuvent être placés en parallèle ou en série réciproquement à l'entrée 2 et/ou à la sortie 3 de l'enceinte 1. Leur disposition en parallèle permet d'augmenter la capacité et la vitesse de chargement de la chaîne 11 de transport des produits.

Le placement de sas 14,15 en série permet d'augmenter graduellement la pression appliquée sur les produits lors de leur engagement dans le compartiment d'entrée 7, en faisant circuler ces produits de sas en sas qui ont alors une pression croissante l'un par rapport à l'autre, ou de diminuer graduellement la pression lors de leur extraction du compartiment de sortie 8. Cette augmentation par pallier de la pression imposée aux produits permet d'éviter d'endommager les emballages, en particulier dans le cas d'emballages souples.

A ce propos, la pression subie par les emballages des produits augmente au fur et à mesure du cheminement de ces derniers au travers des colonnes hydrostatiques 9 depuis l'entrée 2 vers le compartiment intermédiaire 4 tandis qu'elle diminue progressivement depuis le compartiment intermédiaire 4 vers la sortie 3. De cette manière, la température variant proportionnellement par rapport à la pression imposée aux produits et à leurs emballages, l'augmentation graduelle de la pression compense l'effet de l'augmentation de la température, en particulier la dilation du contenu des emballages, rigides, souples ou partiellement souples.

Les sas 14,15 constituent donc, substantiellement et réciproquement, des moyens de chargement et de déchargement en produit des moyens d'acheminement 10 à hauteur des compartiments d'entrée 7 et de sortie 8.

Si ces sas 14,15 peuvent être à double porte, on peut encore imaginer la conception d'un sas en étoile, comme visible sur la figure 4.

Les compartiments d'extrémités 7,8 de l'enceinte 1 ne sont donc plus soumis à la pression atmosphérique fixe mais à une pression variable. La pression du compartiment intermédiaire 4 peut donc être égale à la somme de cette contre-pression et des pressions résultant de chaque colonne hydrostatique 9 d'un compartiment 5,6, et peut, par conséquent, être modulée à tout moment. Cette variation permet la variation de la température dans l'enceinte 1, plus particulièrement dans les compartiments de chauffage 5 et de refroidissement 6.

En Outre, le dispositif comprend des moyens de variation du temps de circulation 16 d'un produit au travers d'un compartiment de l'enceinte 1.

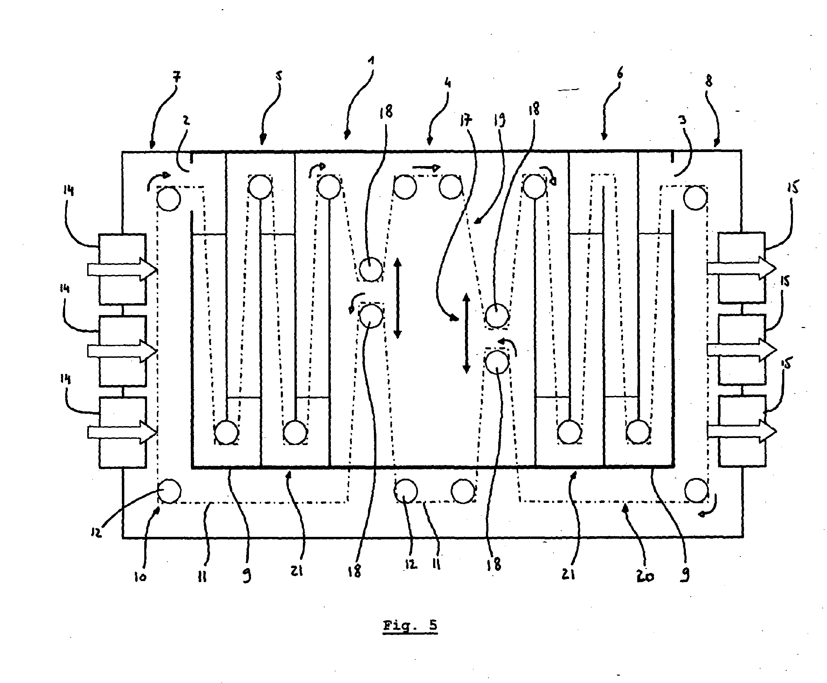

Dans le mode de réalisation particulier visible sur la figure 5, ces moyens de variation du temps de circulation 16 consistent en des moyens de variation 17 de la trajectoire parcourue par lesdits moyens d'acheminement 10, plus particulièrement entre les sas de chargement 13 et de déchargement 14.

Dans une configuration particulière, ces moyens de variation de la trajectoire 17 sont constitués par au moins un rouleau de renvoi mobile 18 possédant un degré de liberté pour rallonger ou, selon le cas, raccourcir la longueur de chaîne 11 dans l'un et/ou l'autre compartiment 4,5,6,7,8.

De manière avantageuse, lesdits moyens de variation de la trajectoire 17 constituent en outre des moyens de commande d'arrêt et/ou de ralentissement de ladite chaîne 11 au devant desdits sas de chargement 14 ou de déchargement 15 sans modifier l'entraînement de ladite chaîne 11.

Sur la figure 5 où un exemple de réalisation est illustré, ce ou ces rouleau de renvoi mobiles 18 sont conçus à même de se déplacer verticalement au niveau du compartiment intermédiaire 4. Le déplacement d'un rouleau mobile 18 vers le haut diminue la longueur de chaîne 11, alors que son déplacement vers le bas l'augmente.

A ce propos, lesdits moyens de variation de la trajectoire 17 sont définis, plus particulièrement, par au moins deux rouleaux de renvois mobiles 18, dont l'un est situé sur le tracé 19 de la chaîne 11 entre l'extrémité d'entrée 2 et l'extrémité de sortie 3 de l'enceinte 1, l'autre étant situé sur le tracé de retour 20 de la chaîne 11 entre le compartiment de sortie 8 et le compartiment d'entrée 7.

Ainsi, en rallongeant, par exemple, le tracé 19 parcouru par la chaîne 11 dans l'enceinte 1, tout en raccourcissant sa trajectoire sur le tracé retour 20, entre le compartiment de sortie 7 et le compartiment d'entrée 8, il en résulte nécessairement un ralentissement de cette chaîne 11, voire l'arrêt, notamment au devant du ou des sas de déchargement 15, favorisant celui-ci. En procédant de manière inverse, on peut obtenir le ralentissement, voire l'arrêt de la chaîne 11, au devant du ou des sas de chargement 14, facilitant le transfert des produits de l'un vers l'autre.

De la même manière, en agissant sur deux rouleaux de renvoi 18 mobiles, disposés à bon escient dans l'enceinte 1, il est possible de déterminer avec précision le temps de transit des produits dans le compartiment de chauffage 5 et/ou le compartiment de refroidissement 6.

Comme il ressort de la description qui précède, cette solution permet d'entraîner la chaîne de transport 11 à vitesse constante par des moyens moteurs appropriés qui ne subissent pas les contraintes dues au ralentissement, à l'arrêt ou à l'accélération de ladite chaîne 11.

De manière tout particulièrement avantageuse, une colonne hydrostatique 9 des compartiments de chauffage 5 et de refroidissement 6 constitue un module 21, de sorte qu'il est aisé d'adapter très facilement le nombre de ces modules qui viennent composer chacun de ces compartiments.

On rappellera, cependant, qu'en raison des particularité de la présente invention, à savoir la présence des compartiments d'entrée 7 et de sortie sous pression 8, et des moyens de variation du temps de circulation 16 d'un produit au travers de l'un quelconque des compartiments 4,5,6,7,8 de l'enceinte 1, et ceci grâce à une gestion de la trajectoire parcourue par ces produits dans ces compartiments, le dispositif de pasteurisation ou stérilisation selon l'invention est en mesure de convenir à une très large gamme de produits moyennant un nombre de modules 21 limité.

## Revendications

1. Procédé de pasteurisation ou de stérilisation de produit, consistant à :
- transférer ledit produit depuis l'extérieur, à la pression atmosphérique, dans un compartiment d'entrée (7) sous pression d'une enceinte (1) au travers d'au moins un sas de mise sous pression (14);
- charger ledit produit sur des moyens d'acheminement (10) assurant leur transfert au travers de ladite enceinte (1) depuis le compartiment d'entrée (7) vers un compartiment de sortie (8) ;
- chauffer le produit en le plongeant dans des colonnes hydrostatiques (9) ;
- faire traverser audit produit un compartiment intermédiaire (4) de mise sous pression ;
- refroidir le produit en le plongeant dans des colonnes hydrostatiques (9) ;
- décharger ledit produit des moyens d'acheminement (10) au niveau d'un compartiment de sortie (8) sous pression ;
- transférer vers l'extérieur ledit produit depuis le compartiment de sortie (8) au travers d'au moins un sas (15) de mise à la pression atmosphérique ;
**caractérisé en ce qu'**il consiste à faire varier le temps de passage du produit à l'intérieur de ladite enceinte (1) en faisant varier la longueur de la trajectoire suivie de ces produits dans cette enceinte (1).

2. Dispositif de mise en oeuvre du procédé précédent, constitué d'une enceinte (1) pourvue, d'une part, sous forme de colonnes hydrostatiques (9), d'un compartiment de chauffage (5) et d'un compartiment de refroidissement (6) reliés par un compartiment intermédiaire (4) de mise sous pression, ledit dispositif comportant encore des moyens d'acheminement (10) des produits depuis une extrémité d'entrée (2) vers une extrémité de sortie (3) de l'enceinte (1), ladite enceinte (1) comportant un compartiment d'entrée (7) et un compartiment de sortie (8) munis de moyens de mise sous pression (13) et équipés respectivement, d'au moins un sas (14) de mise sous pression des produits et d'au moins un sas (15) de mise à la pression atmosphérique des produits, characterisé par le fait que ladite enceinte comporte des moyens de variation du temps de circulation (16) d'un produit au travers de l'un quelconque des compartiments (4,5,6,7,8) de l'enceinte (1), lesdits moyens de variation du temps de circulation consistant en des moyens de variation (17) de la trajectoire parcourue par lesdits moyens d'acheminement (10) entre les sas de chargement (14) et de déchargement (15).

3. Dispositif selon la revendication 2, **caractérisé en qu'**il comporte plusieurs sas (14,15), du type à deux portes ou en étoile, raccordés en parallèle et/ou en série aux compartiments d'entrée (7) et/ou de sortie (8).

4. Dispositif selon l'une quelconque des revendications 2 ou 3, **caractérisé en ce que** le ou les sas (14,15) constituent, substantiellement, des moyens de chargement et de déchargement en produit des moyens d'acheminement (10) à hauteur des compartiments d'entrée (7) et de sortie (8).

5. Dispositif selon l'une quelconque des revendications 2 à 4, **caractérisé en ce que** lesdits moyens d'acheminement comportent une chaîne (11) et **en ce que** lesdits moyens de variation de la trajectoire (17) constituent des moyens de commande d'arrêt et/ou de ralentissement de ladite chaîne (11) au devant desdits sas de chargement (14) ou de déchargement (15).

6. Dispositif selon l'une quelconque des revendications 2 à 5, **caractérisé en ce que** lesdits moyens d'acheminement comportent une chaîne (11) et **en ce que** les moyens de variation de la trajectoire (17) sont constitués par au moins un rouleau de renvoi mobile (18) possédant un degré de liberté pour rallonger ou raccourcir la longueur de chaîne (11) dans l'un et/ou l'autre compartiment (4,5,6,7,8).

7. Dispositif selon l'une quelconque des revendications 2 à 6, **caractérisé en ce que** lesdits moyens d'acheminement comportent une chaîne (11) et **en ce que** lesdits moyens de variation de la trajectoire (17) sont définis par au moins deux rouleaux de renvois mobiles (18), dont l'un est situé sur le tracé (19) de la chaîne (11) entre l'extrémité d'entrée (2) et l'extrémité de sortie (3) de l'enceinte (1), l'autre étant situé sur le tracé de retour (20) de la chaîne (11) entre le compartiment de sortie (8) et le compartiment d'entrée (7).

8. Dispositif selon l'une quelconque des revendications 2 à 7, **caractérisé en ce que** les compartiments d'entrée (7) et de sortie (8) sont constitués de modules (21), lesdits modules étant constitués d'au moins une colonne hydrostatique (9).

## Claims

1. Method for pasteurizing or sterilizing products, consisting in:
- transferring said product from outside, under atmospheric pressure, into an inlet compartment (7) under pressure of a chamber (1) through at least one pressurizing airlock (14);
- loading said product onto conveying means (10) ensuring its transfer through said chamber (1) from the inlet compartment (7) to an outlet compartment (8);
- heating the product by plunging it into hydrostatic columns (9);
- causing said product to pass through an intermediate pressurizing compartment (4);
- cooling the product by plunging it into hydrostatic columns (9);
- unloading said product from the conveying means (10) at the level of an outlet compartment (8) under pressure;
- transferring said product from the outlet compartment (8) to the outside through at least one airlock (15) for putting under atmospheric pressure;
which method consists in causing the time of staying of the product inside said chamber (1) to vary by causing the length of the path followed by these products in this chamber (1) to vary.

2. Device for implementing the preceding method, consisting of a chamber (1) provided, on the one hand, in the form of hydrostatic columns (9), with a heating compartment (5) and with a cooling compartment (6), which are connected by means of an intermediate pressurizing compartment (4), said device also including means for conveying (10) the products from an inlet end (2) to an outlet end (3) of the chamber (1), said chamber (1) including an inlet compartment (7) and an outlet compartment (8) provided with pressurizing means (13) and provided with at least one air lock (14) for pressurizing the products and at least one airlock (15) for putting the products under atmospheric pressure, respectively, wherein said chamber includes means for varying the time of circulation (16) of a product through any one of the compartments (4, 5, 6, 7, 8) of the chamber (1), said means for varying the time of circulation consisting of means (17) for causing the path followed by said conveying means (10) between the loading airlock (14) and the unloading airlock (15) to vary.

3. Device according to claim 2, including several airlocks (14, 15) of the type with two doors or star-shaped, connected in parallel and/or in series to the inlet (7) and/or outlet compartments (8).

4. Device according to any of claims 2 or 3, wherein the airlock or airlocks (14, 15) substantially form means for loading with products and unloading the conveying means (10) at the level of the inlet (7) and outlet (8) compartments.

5. Device according to any of claims 2 to 4, wherein said conveying means include a chain (11) and said means for varying the path (17) form means for controlling the stoppage and/or slowing down of said chain (11) in front of said loading (14) or unloading (15) airlocks.

6. Device according to any of claims 2 to 5, wherein said conveying means include a chain (11) and the means for varying the path (17) are formed by at least one movable return pulley (18) having a degree of freedom in order to lengthen or shorten the length of the chain (11) in either compartment (4, 5, 6, 7, 8).

7. Device according to any of claims 2 to 6, wherein said conveying means include a chain (11) and said means for varying the path (17) are formed by at least two movable return pulleys (18), one of which is located on the path (19) of the chain (11) between the inlet end (2) and the outlet end (3) of the chamber (1), the other one being located on the return path (20) of the chain (11) between the outlet compartment (8) and the inlet compartment (7).

8. Device according to any of claims 2 to 7, wherein the inlet (7) and outlet (8) compartments are formed by modules (21), said modules being formed by at least one hydrostatic column (9).

## Patentansprüche

1. Verfahren zur Pasteurisierung oder Sterilisierung von Produkten, darin bestehend:
- das besagte Produkt bei atmosphärischem Druck von außen in eine unter Druck gesetzte Eingangskammer (7) eines Raumes (1) durch wenigstens eine Druckschleuse (14) zu überführen;
- mit dem besagten Produkt Beförderungsmittel (10), die seine Weiterbeförderung durch den besagten Raum (1) von der Eingangskammer (7) in Richtung auf eine Ausgangskammer (8) sichern, zu beschicken;
- das Produkt zu erhitzen, indem es in hydrostatische Säulen (9) eingetaucht wird;
- das besagte Produkt durch eine Zwischenkammer (4) zum Setzen unter Druck passieren zu lassen;
- das Produkt abzukühlen, indem es in hydrostatische Säulen (9) eingetaucht wird;
- das besagte Produkt von den Beförderungsmitteln (10) im Bereich einer unter Druck gesetzten Ausgangskammer (8) zu entnehmen;
- das besagte Produkt von der Ausgangskammer (8) durch zumindest eine Druckschleuse (15) zum Setzen unter Atmosphärendruck nach außen weiterzubefördern;
**dadurch gekennzeichnet, dass** es darin besteht, die Zeit für den Durchlauf des Produktes im Innern des besagten Raumes (1) variieren zu lassen, indem die Länge der von diesen Produkten in diesem Raum (1) gefolgten Bahn verändert wird.

2. Vorrichtung zum Ausführen des vorgehenden Verfahrens, bestehend aus einem Raum (1), ausgestattet, einerseits, in der Form von hydrostatischen Säulen (9), mit einer Erhitzungskammer (5) und mit einer Abkühlkammer (6), die durch eine Zwischenkammer (4) zum Setzen unter Druck mit einander verbunden sind, wobei die besagte Vorrichtung noch Mittel zur Beförderung (10) der Produkte von einem Eingangsende (2) in Richtung auf ein Ausgangsende (3) des Raumes (1) umfasst, wobei der besagte Raum (1) eine Eingangskammer (7) und eine Ausgangskammer (8) umfasst, die mit Mitteln zum Setzen unter Druck (13) ausgestattet sind und respektive mit wenigstens einer Druckschleuse (14) zum Setzen der Produkte unter Druck bzw. mit wenigstens einer Druckschleuse (15) zum Setzen der Produkte unter Atmosphärendruck ausgerüstet sind, **dadurch gekennzeichnet, dass** der besagte Raum Mittel zur Änderung der Zeit für den Umlauf (16) eines Produktes durch eine beliebige von den Kammern (4, 5, 6, 7, 8) des Raumes (1) umfasst, wobei die besagten Mittel zur Änderung der Umlaufzeit aus Mitteln zur Änderung (17) der von den besagten Beförderungsmitteln (10) zwischen den Druckschleusen zur Beschickung (14) und Entleerung (15) zurückgelegten Bahn bestehen.

3. Vorrichtung nach Anspruch 2, **dadurch gekennzeichnet, dass** sie mehrere Druckschleusen (14, 15) der Art mit zwei Türen oder sternförmig umfasst, die parallel und/oder in Reihenschaltung an den Eingangs- (7) und/oder Ausgangskammern (8) angeschlossen sind.

4. Vorrichtung nach irgendeinem der Ansprüche 2 oder 3, **dadurch gekennzeichnet, dass** die Druckschleuse oder -schleusen (14, 15) im wesentlichen Mittel zur Beschickung der Beförderungsmittel (10) mit Produkt und zur Entleerung des Produkts im Bereich der Eingangs- (7) und Ausgangskammern (8) bilden.

5. Vorrichtung nach irgendeinem der Ansprüche 2 bis 4, **dadurch gekennzeichnet, dass** die besagten Beförderungsmittel eine Kette (11) umfassen und dass die besagten Mittel zur Änderung der Bahn (17) Mittel zum Steuern der Bremsung und/oder der Verlangsamung der besagten Kette (11) gegenüber den besagten Druckschleusen zur Beschickung (14) oder Entleerung (15) bilden.

6. Vorrichtung nach irgendeinem der Ansprüche 2 bis 5, **dadurch gekennzeichnet, dass** die besagten Beförderungsmittel eine Kette (11) umfassen und dass die Mittel zur Änderung der Bahn (17) durch zumindest eine bewegliche Lenkrolle (18) gebildet sind, die einen Freiheitsgrad besitzt, um die Länge der Kette (11) in der einen und/oder anderen Kammer (4, 5, 6, 7, 8) zu verlängern oder zu verkürzen.

7. Vorrichtung nach irgendeinem der Ansprüche 2 bis 6, **dadurch gekennzeichnet, dass** die besagten Beförderungsmittel eine Kette (11) umfassen und dass die besagten Mittel zur Änderung der Bahn (17) durch zumindest zwei bewegliche Lenkrollen (18) gebildet sind, von denen die eine auf der Bahn (19) der Kette (11) zwischen dem Eingangsende (2) und dem Ausgangsende (3) des Raumes (1) befindlich ist, wobei die andere auf der Rückkehrbahn (20) der Kette (11) zwischen der Ausgangskammer (8) und der Eingangskammer (7) befindlich sei.

8. Vorrichtung nach irgendeinem der Ansprüche 2 bis 7, **dadurch gekennzeichnet, dass** die Eingangs- (7) und Ausgangskammern (8) durch Module (21) gebildet sind, wobei die besagten Module durch wenigstens eine hydrostatische Säule (9) gebildet seien.
